# EUROPEAN PATENT SPECIFICATION

(11) **EP 0 626 016 B1**
(45) Date of publication and mention of the grant of the patent: **03.06.1998**
(21) Application number: 93903259.5
(22) Date of filing: 10.02.1993
(51) Int. Cl.: C12Q 1/28

(54) **CHEMILUMINESCENT ENHANCERS**
CHEMILUMINESZENZVERSTÄRKER
STIMULATEURS DE CHIMIOLUMINESCENCE

(30) Priority: 10.02.1992 GB 9202769; 07.08.1992 GB 9216784
(43) Date of publication of application: 30.11.1994
(73) Proprietor: BRITISH TECHNOLOGY GROUP LIMITED, London EC4M 7SB (GB)
(72) Inventor: KRICKA, Larry, Jan, Berwyn, PA 19312 (US)
(74) Representative: Percy, Richard Keith
(86) International application number: GB9300271
(87) International publication number: WO9316195

(56) References cited:
- WO-A-91/05872
- GB-A- 2 162 946
- METHODS IN ENZYMOLOGY vol. 133, 1986, NEW YORK (US) pages 331 - 353 GARY H. THORPE AND LARRY J. KRICKA 'Enhanced Chemiluminescent Reactions Catalyzed by Horseradish Peroxidase'
- CLINICAL CHEMISTRY. vol. 37, no. 9, September 1991, WINSTON US pages 1472 - 1481 LARRY J. KRICKA 'Chemiluminescent and Bioluminescent Techniques' cited in the application

## Description

### Background of the invention

### 1. Field of the invention

The present invention relates to an enhanced chemiluminescent reaction especially for use in a diagnostic assay, particularly immunoassay, and to a diagnostic kit for use in the assay. A chemiluminescent reaction is a chemical reaction which results in the emission of light. The luminescent emission is generally of sufficient duration to enable the light emitted to be detected or measured, and thereby to allow the detection or quantification of an analyte. The chemiluminescent reaction with which this invention is concerned is that between a 2,3-dihydro-1,4-phthalazinedione (DPD), especially luminol, with an oxidant, especially hydrogen peroxide, and a peroxidase enzyme, especially horseradish peroxidase, which catalyses the oxidation of the DPD by the oxidant. The oxidation is accompanied by emission of light.

### 2. Description of the prior art

Luminescent assays making use of the above-mentioned peroxidase-catalysed oxidation of a DPD include several types. This invention is concerned primarily with those in which the presence or amount of peroxidase is determined. It includes predominantly assays wherein horseradish peroxidase is conjugated to a ligand in order to label it and a luminescent reaction is used to detect or quantitate the label. This category includes ELISAs, competitive EIAs and nucleic acid hybridization assays, based on peroxidase labels. However, assays for measurement of free peroxidase, e.g. for analytical purposes, are also included.

A review of luminescent assays has been published by L. J. Kricka, Clinical Chemistry 37, 1472-1481 (1991).

The sensitivity of the peroxidase-catalysed chemiluminescent oxidation of DPDs can be enhanced by including in the reagents an enhancer, namely a 6-hydroxybenzothiazole (European Patent No. 87959B), a phenol selected from a narrowly defined class (European Patent No. 116454B or U.S. Patent No. 4,598,044), or an aromatic amine selected from a narrowly defined class (U.K. Patent No. 2162946B or U.S. Patent No. 4,729,950). A further class of substituted phenols that enhance chemiluminescent reactions of this type are phenols substituted in ortho and/or para positions by imidazolyl or benzimidazolyl (U.K. Patent No. 2205945B, European Patent No. 296752B). These patents are owned by National Research Development Corporation. European Patent Application Publication No. 219352A (Minnesota Mining and Mfg. Co.) describes various aromatic amines, including some of those previously mentioned in U.K. Application 2162946A, as enhancers. It is an object of the invention to extend the range of effective enhancers. This is a difficult task because no theory or mechanism has been published to explain how one should attempt to select candidate compounds to try as enhancers. For the purposes of the present application the term "enhancer" and related terms will be used to include compounds that increase the total light output and/or the signal:background ratio of a chemiluminescent assay, at at least one concentration of compound.

### Summary of the invention

It has now been found that certain organoboron compounds are effective enhancers of chemiluminescence in a reaction between a dihydrophthalazinedione (DPD), a peroxidase enzyme catalyst and an oxidant. The organoboron enhancers used in the present invention are compounds of formula (I) 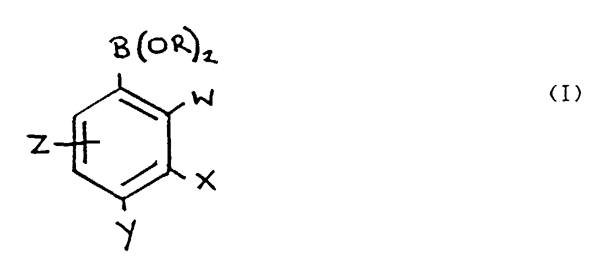 in which the R groups are the same and each is selected from hydrogen, n-butyl, 4'-chlorophenyl and 3',5'-dichlorophenyl; or the Rs together are 0,0-propylene (thereby forming with the boron atom, a cyclic ether);
W is selected from hydrogen methyl, methoxy, hydroxy and chloro;
X is selected from hydrogen, chloro, amino and nitro;
Y is selected from hydrogen, methyl, carboxy, chloro, bromo, iodo, phenyl, phenoxy, 4'-chloroanilino, 4'-boronylphenyl, 4'-bromophenyl, 2'-carboxyethenyl and trimethylsilyl;
Z is selected from hydrogen, 5-chloro, 5-bromo, 5-(3'-trifluoromethyl)phenylazo and 6-chloro; or
W and X together may represent a fused benzene ring and X and Y together may represent a fused benzene ring substituted by hydroxy in the 6-position of the naphthalene ring numbering, provided that

(1) when each R is hydrogen:
   (a) W, X, Y, Z are each hydrogen; or
   (b) W, X and Z are each hydrogen and Y is selected from iodo, bromo, chloro, trimethylsilyl, phenoxy, phenyl, 4'-chloroanilino, methyl, 4'-boronylphenyl and 2'-carboxyethenyl; or
   (c) W and Z are each hydrogen and:
      (i) X and Y together represent a fused benzene ring substituted by hydroxy in the 6-position of the naphthalene ring numbering; or
      (ii) X is either nitro or chloro and Y is chloro; or
      (iii) X is nitro and Y is carboxy; or
   (d) W, Y and Z are each hydrogen and X is amino, chloro or nitro; or
   (e) W and X together represent a fused benzene ring and Y and Z are each hydrogen; or
   (f) X and Y are each hydrogen and :
      (i) W is methoxy and Z is 5-bromo; or
      (ii) W is hydroxy and Z is 5-(3'-trifluoromethyl)phenylazo; or
      (iii) W is methyl and Z is hydrogen; or
      (g) W is chloro, X is chloro and Y and Z are each hydrogen; or
      (h) W and Y are each chloro, X is amino and Z is 6-chloro;
(2) when each R is n-butyl, W, X and Z are each hydrogen and Y is bromo or 4'-bromophenyl;
(3) when each R is 4'-chlorophenyl, W, X and Z are each hydrogen and Y is chloro;
(4) when each R is 3',5'-dichlorophenyl, W and Y are each hydrogen, X is chloro and Z is 5-chloro; and
(5) when the Rs together represent O,O-propylene, X, Y and Z are each hydrogen; or is selected from the compounds bis(catechol)borate, boroglycine, pentaerythritol borate, 4-(3'-borono-4'-hydroxyphenylazo)benzoic acid, diphenylisobutoxyborane; diphenylboronic anhydride and dimethylphenylboronic acid.

Of the compounds listed above those with 0-alkyl groups may undergo spontaneous hydrolysis, e.g. diphenylisobutoxyborane, 4-(4'-bromophenyl)phenyl-di-n-butoxyborane or 4-bromophenyl-di-n-butoxyborane. Others containing the structure (Aryl-O)₂-B-Aryl may also undergo spontaneous hydrolysis, e.g. di(3',5'-di-chlorophenoxy)-3,5-dichlorophenylborane or 4-chlorophenyl-di-(4'-chlorophenoxy)borane.

The enhancers of the present invention that fall into formula (I) may be easily looked at by means of the table below in which H is hydrogen and the key to the compound reference numbers is present in the Examples.

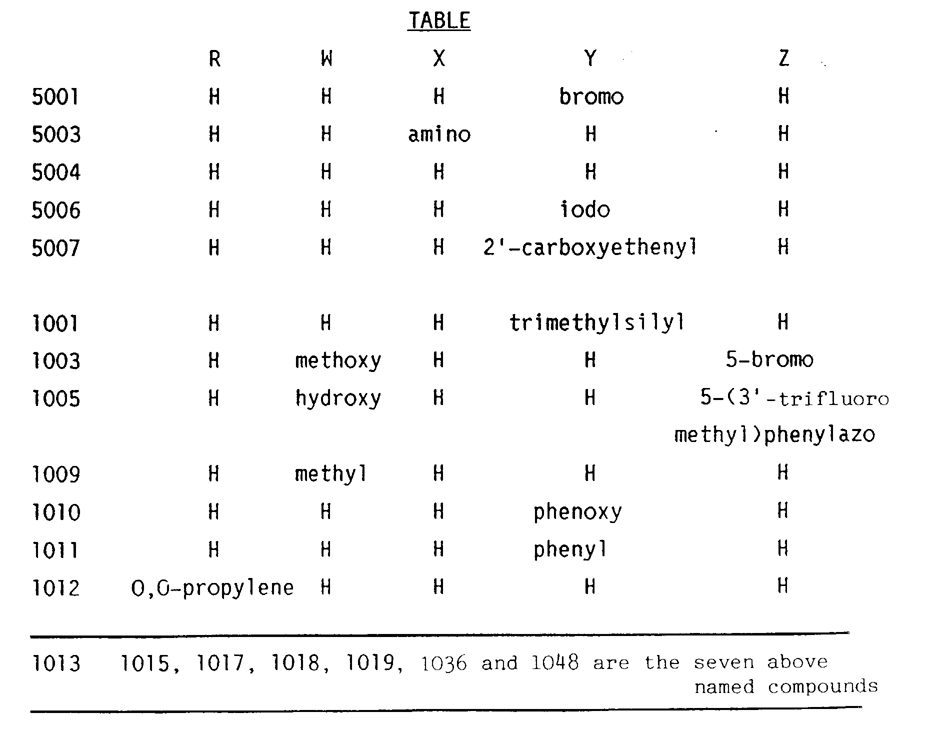

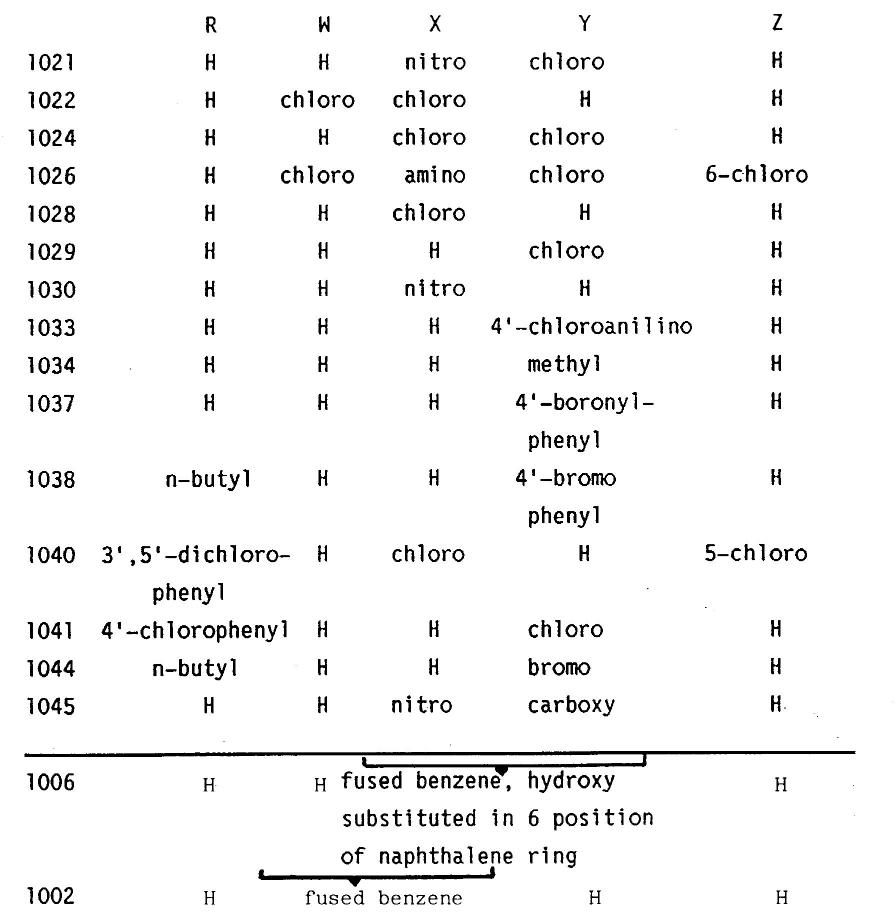

While the invention applies to increasing the light output and/or the signal:background ratio from any chemiluminescent reaction involving the above-stated reaction partners, for any purpose, it is primarily of interest in connection with an assay. The term "assay" herein covers detection, semi- quantitation and quantitation. Typically, the assay is carried out so that the light output is relatable to the amount of peroxidase employed, the peroxidase then being the substance directly determined. The ratio of light output when peroxidase is present in the sample to light output when it is absent becomes important in assuring the sensitivity of the assay. This is conveniently termed a "signal to background" ratio. Similarly, if the substance to be determined is another of the reaction partners, the "signal" denotes the presence of the substance to be determined, the "background" its absence.

Although the invention is usable to determine the presence or amount of any one of the four above-stated reaction partners, such a reaction partner is not necessarily itself the substance to be assayed. Thus, the oxidant can be produced as a result of an earlier reaction or cascade of earlier reactions carried out on a sample. The peroxidase or the luminol can be in the form of a conjugate to, say, an antibody which is used in an immunoassay to determine an antigen. The invention is accordingly applicable to any method of diagnostic assay of a substance, the presence or amount of which is relatable to the presence or amount of a reaction partner selected from the group consisting of a DPD, a peroxidase enzyme, an oxidant and an enhancer which together are reactable in a chemiluminescent reaction and wherein the reaction is carried out, the light output is detected or measured and thence the presence or amount of the substance to be assayed is related to the light output.

The invention also includes a kit for use in the assay comprising the DPD, the peroxidase and the enhancer. The oxidant could be supplied separately or included in the kit.

### Brief description of the drawings

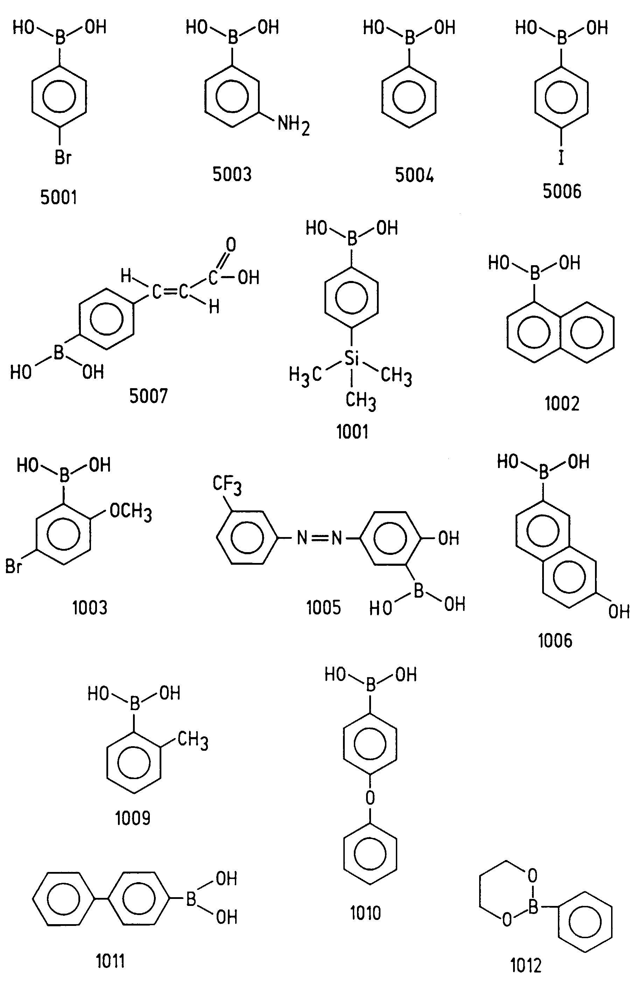

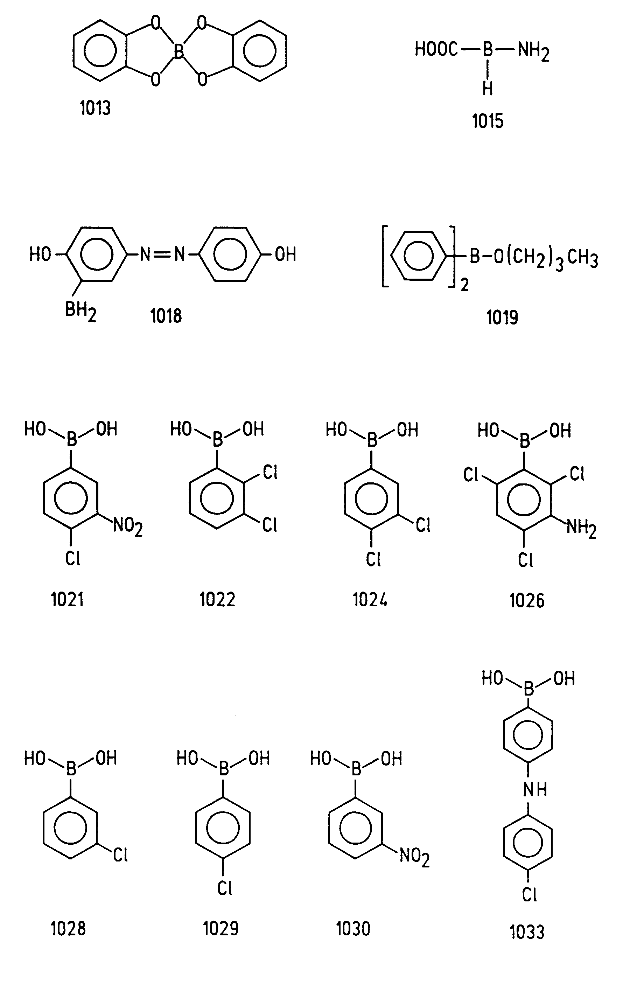

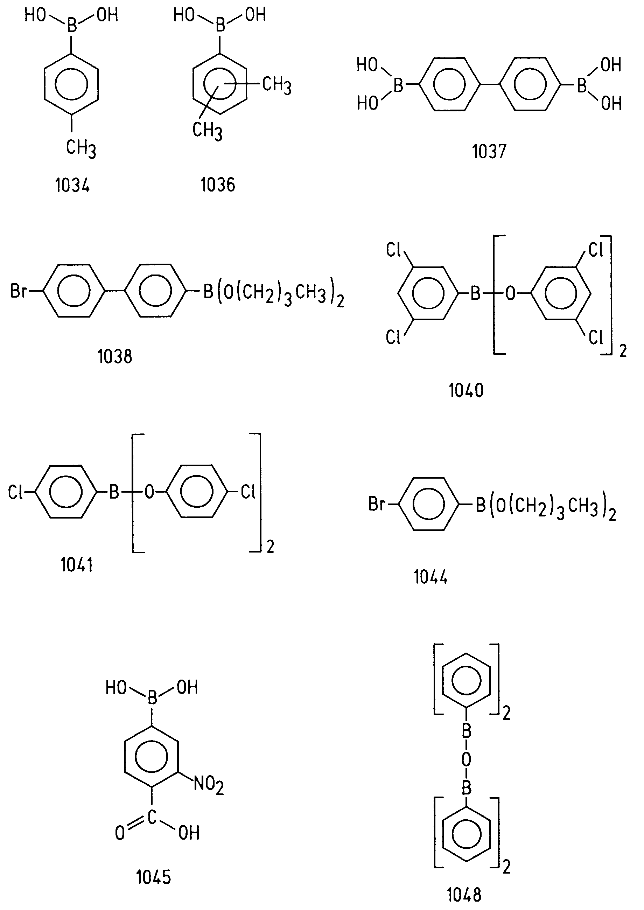

The Figure shows the structure of some of the enhancers of the present invention.

### Description of the preferred embodiments

The enhancers of the present invention include 4-iodophenylboronic acid (PIBA), 4-bromophenylboronic acid (PBBA), 4-chlorophenylboronic acid, 3-chlorophenylboronic acid, 3,4-dichlorophenylboronic acid, 2,3-dichlorophenylboronic acid, 5-bromo-2-methoxybenzeneboronic acid, 3-nitrophenylboronic acid, 4-chloro-3-nitrophenylboronic acid, 3-aminophenylboronic acid, 3-amino-2,4,6-trichlorophenylboronic acid, 4-(2'-carboxyethenyl)phenylboronic acid, 1-naphthaleneboronic acid, 6-hydroxy-2-naphthaleneboronic acid, phenylboronic acid, 2-methylphenylboronic acid, 4-methylphenylboronic acid, dimethyl-phenylboronic acid, 4-bromophenyl-di-n-butoxyborane, 4-carboxy-3-nitrophenylboronic acid, 4-(trimethylsilyl)benzeneboronic acid, 4-biphenylboronic acid, 4-(phenoxy)benzeneboronic acid, 4-(3'-borono-4'-hydroxyphenylazo)benzoic acid, diphenylisobutoxyborane, 4-(4' -chloro-anilino)phenylboronic acid, 4,4'-bis(phenylboronic acid), 4-(4'-bromophenyl)phenyl-di-n-butoxyborane, di(3',5'-dichlorophenoxy)-3,5-dichlorophenylborane, 4-chlorophenyl-di-(4'-chlorophenoxy)borane, pentaerythritol borate, boroglycine, 2-phenyl-1,3,2-dioxaborinane, bis(catechol)borate and 2-hydroxy-5-[(3'-trifluoromethyl)phenylazo]benzeneboronic acid and diphenylboronic anhydride.

The preferred enhancers are PIBA, PBBA, 4-biphenylboronic acid, 4-(trimethylsilyl)-benzeneboronic acid, boroglycine, 2-hydroxy-5-[(3'trifluoromethyl)phenylazo]benzeneboronic acid, 4-chloro-3-nitrophenylboronic acid, 4-chlorophenylboronic acid; 4-(2'-carboxyethenyl)phenylboronic acid, 4-(4'-bromophenyl)phenyl-di-n-butoxyborane, 4-chlorophenyl-di-(4'-chlorophenoxy)borane, 4,4'-bis(phenylboronic acid), diphenylboronic anhydride, 4-(4'-chloro-anilino)phenylboronic acid and 4-bromophenyl-di-n-butoxyborane as they increase light output as well as reducing the background luminescence. The remaining enhancers exert their effect primarily by reducing the background luminescence, and thereby improving the signal:background ratio.

The structures of some of these compounds are shown in the accompanying Figure. The reference numbers are explained in the Examples.

The improvement in signal:background ratio is of importance in controlling the sensitivity of chemiluminescent assays. The enhancers of the present invention are therefore of particular use in those situations where a high degree of sensitivity is required, for example in blotting assays. Thus the present invention is of especial use in blotting assays including Western, Southern and Northern blotting assays, as well as dot blots and other nucleic acid hybridisation assays.

The best results are obtained at higher pH. Preferably the pH is in the range 7.5 to 9 at the time of mixing all the reagents.

Any chemiluminescent DPD can be used in the invention, that is to say any DPD which is oxidisable in the presence of a peroxidase catalyst by an added oxidant to give chemiluminescence can be used. Examples are luminol, isoluminol, ABEI and AHEI, and 7-dimethylaminonaphthalene-1,2-dicarboxylic acid hydrazide, of which luminol is normally preferred. The DPD can be free or conjugated to a ligand to provide a direct label. Such luminophore-labelled assays are known in the art.

The oxidant can be any added substance (not oxygen itself) which oxidises the DPD in a light-emitting reaction; hydrogen peroxide is usual, but a perborate, such as the sodium salt, is an alternative.

The peroxidase enzyme will normally be HRP and of a grade appropriate to use in luminescent assays. Preferably the HRP is a basic isoenzyme, for example of Sigma Type VIA or IX. It can be free or conjugated to a ligand.

The concentrations of the reaction partners of the chemiluminescent reaction will depend on the nature of the assay being carried out and particularly on which of them is being assayed. Generally stated, the light output is greater, the greater the concentration of DPD. Thus, when peroxidase or oxidant is being assayed, the use of excess DPD is recommended. Generally stated, the DPD concentration is desirably from 0.5 micromole to 200 millimoles per litre, preferably 0.5 to 100 micromoles/litre. Generally stated, the oxidant concentration is desirably in the range 0.5 micromoles to 300 millimoles/ litre, preferably 10 to 200 millimoles/litre.

The concentration of peroxidase is of interest if peroxidase is not the reaction partner being assayed. Excess peroxidase does not normally have a marked effect on light intensity, the peroxidase being a catalyst which is recycled. Where luminol or the oxidant is being assayed, therefore, the peroxidase need only be present in a modest concentration, such as 0.01 microgram to 5000 mg./litre, preferably not more than 50 mg./litre, but depending on the activity of the peroxidase per gram.

The concentration of the enhancer will usually be in the range 0.01 micromole to 4 moles/litre, preferably 10 micromoles to 100 millimoles/litre. It is believed that the enhancer or a species or derivative thereof competes with the DPD in the reaction and it is therefore desirable to use a considerable excess of DPD relative to the enhancer, preferably between 2 and 20 times the molar concentration of the enhancer.

In brief, all conditions and features of the chemiluminescent reactions, the reaction partners thereof, applications of the assay and so on (except where inconsistent with the above description) can be as set forth in European Patent No. 116454B.

The following Examples illustrate the invention. In the Examples, compounds of series 5000 are obtainable from the stated sources, compounds of series 1000 are identified by means of their Chemical Abstracts Registry Number and others are obtainable from US Borax Research Corporation, 412 Crescent Way, Anaheim, California 92901-9794.

### EXAMPLE 1

This Example shows that para-bromophenylboronic acid (PBBA) enhances a chemiluminescent reaction between luminol (LU) horseradish peroxidase (HRP), and H₂O₂ giving a high signal: background ratio.

### 1. Effect of PBBA (Compound 5001) on the signal :background ratio of a chemiluminescent reaction

PBBA was added in various concentrations (range 0.05µg to 0.5µg) to a luminol-H₂O₂ reaction in the presence and absence of HRP. The luminol-hydrogen peroxide reagent was prepared as follows: sodium luminol (12.5mg) was dissolved in 50ml of Tris buffer (0.1mol/l, pH 8.6), and 15.5µl of hydrogen peroxide (30% w/v) was mixed with 0.5ml of Tris buffer (0.lmol/l, pH 8.6). These two solutions were combined and protected from light. The luminol-hydrogen peroxide reagent (100µl of a 1:10 dilution) was added to a cuvette together with either 10µl of HRP Type VI A (HRP, Sigma Chemical Co., 1:50,000 dilution) or as a control 10µl of Tris Buffer (0.1mol/l, pH 8.6), and various amounts of PBBA (Aldrich Chemical Co.), range 0.05µg-0.5µg in 0.1mol/l Tris, pH 8.6). The reagents were mixed and the light emission recorded after 5 minutes on a Berthold Biolumat LB9500C.

This experiment was repeated, replacing the luminol with isoluminol. The measured light output and signal:background ratios are shown in Table 1. Column (a) shows that PBBA enhances the light emission in the luminol-HRP-peroxidase reaction. Column (b) shows that addition of PBBA reduces the background level of luminescence. Columns (d) and (e) show a similar effect with isoluminol.

**TABLE 1**

| | Luminol | | | Isoluminol | | |
|---|---|---|---|---|---|---|
| | (a) | (b) | (c) | (d) | (e) | (f) |
| PBBA (µg) | Signal (with HRP) | Signal (no HRP) | Signal: Background ratio | Signal (with HRP) | Signal (no HRP) | Signal: Background ratio |
| 0 | 87,616 | 18,833 | 4.7 | 14,485 | 7927 | 1.8 |
| 0.05 | 101,227 | 16,556 | 6.1 | 15,772 | 7664 | 2.1 |
| 0.1 | 108,213 | 16,065 | 6.7 | 16,553 | 6114 | 2.7 |
| 0.2 | 112,644 | 13,890 | 8.1 | 68,282 | 5736 | 11.9 |
| 0.3 | 122,116 | 10,899 | 11.2 | 619,880 | 5613 | 110.4 |
| 0.4 | 133,401 | 10,706 | 12.5 | 775,965 | 5278 | 147.0 |
| 0.5 | 151,572 | 8,963 | 16.9 | | | |

### 2. Effect of PBBA on the light output of a chemiluminescent reaction

The above experiment was repeated this time keeping the amount of PBBA enhancer fixed and varying the amount of HRP. The reagents used were 100µl of luminol-hydrogen peroxide, either (a) 40µl of PBBA (0.01mg/ml in 0.lmol/l Tris buffer, pH 8.6) or (b) as a control, 40µl of Tris buffer (0.1mol/l, pH 8.6) and various amounts of HRP (range 5µl to 40µl of 1:50,000 dilution of a lmg/ml stock solution).

The reagents were mixed and light output recorded after 5 minutes on a Berthold Biolumat LB9500C. The measured signal (light output) and signal:background ratios are shown in Table 2. By comparing columns (a) and (b) it can be seen that PBBA increased the light output of the chemiluminescent reaction at all concentrations tested. An improved signal:background ratio is shown by comparing results with HRP present with the zero HRP results.

**TABLE 2**

| | (a) | | (b) | |
|---|---|---|---|---|
| HRP (pg) | Signal (with PBBA) | Signal: Background Ratio | Signal (no PBBA) | Signal: Background Ratio |
| 0 | 9,125 | | 13,405 | |
| 100 | 15,861 | 1.7 | 15,148 | 1.1 |
| 200 | 22,895 | 2.5 | 19,384 | 1.4 |
| 400 | 38,495 | 4.2 | 25,968 | 1.9 |
| 600 | 61,491 | 6.7 | 33,964 | 2.5 |
| 800 | 105,972 | 11.6 | 34,790 | 2.6 |

### EXAMPLE 2

### Effect of organoboron compounds on a chemiluminescent reaction

A solution of 2,4-dichlorophenylboronic acid (5002) (1mg/ml, Lancaster Synthesis Inc., Windham, NH) was prepared as follows: 5mg 2,4-dichlorophenylboronic acid was dissolved in 50µl of DMSO, then added to 4950µl of Tris buffer (0.1mol/l, pH 8.6). Solutions of 3-aminophenylboronic acid (5003) (1mg/ml, Sigma), 3-nitro-phenylboronic acid (lmg/ml, Aldrich), phenylboronic acid (5004) (1mg/ml, Aldrich) and butaneborinic acid (5005) (1mg/ml, Sigma) were prepared in Tris buffer (0.1mol/l, pH 8.6). The stock solution of HRP Type VIA (1mg/ml) and luminol-hydrogen peroxide reagent were prepared as described previously. Luminol-hydrogen peroxide (100µl, 1:10 dilution) was mixed with either 10µl of HRP (1:50,000 dilution) or, 10µl of Tris buffer (0.1mol/l, pH 8.6). The light emission was measured in a Berthold Biolumat LB9500C, and then 5µl of 2,4-dichlorophenylboronic acid was added and the light emission was remeasured. A control without any test compound was run in parallel.

The above experiment was repeated except that 2,4-dichlorophenylboronic acid was replaced by 3-aminophenylboronic acid (5µl), 3-nitrophenylboronic acid (5µl), phenylboronic acid (5µl), or butaneborinic acid (40µl).

The measured light output and signal:background ratios are shown in Table 3 for each compound tested and its control. The control consisted of buffer in place of the compound under test as an enhancer. None of the compounds tested increased the light emission from the HRP catalyzed oxidation of luminol. However, the signal to background ratio was improved in the case of 3-nitrophenylboronic acid, phenylboronic acid, and 3-aminophenylboronic acid as compared to their control values. This was due to the reduction in the background light emission from the luminol-peroxide reagent caused by these compounds. Thus, these three compounds are of use in the present invention. 2,4-dichlorophenylboronic acid and butaneborinic acid are not of use as they neither increased the light output or the signal: background ratio. The small increase in light output observed for butaneborinic acid is not significantly relevant to be of use in the present invention.

**TABLE 3**

| | | Signal (with HRP | Signal (no HRP | Signal: Background Ratio |
|---|---|---|---|---|
| 2,4-dichlorophenylboronic acid (5002) | control | 271,566 | 20,990 | 12.9 |
| | test | 128,274 | 16,246 | 7.9 |
| | | | | |
| 3-nitrophenylboronic acid | control | 175,440 | 25,196 | 6.9 |
| | test | 132,887 | 16,400 | 8.1 |
| | | | | |
| phenylboronic acid (5004) | control | 156,658 | 26,564 | 5.9 |
| | test | 54,554 | 5,601 | 9.7 |
| | | | | |
| 3-aminophenylboronic acid (5003) | control | 249,204 | 19,615 | 12.7 |
| | test | 60,222 | 1,038 | 58.0 |
| | | | | |
| butaneborinic acid (5005) | control | 64,652 | 39,640 | 1.6 |
| | test | 65,197 | 54,389 | 1.2 |

### EXAMPLE 3

### Screening of further organoboron compounds

The stock solutions of HRP Type VIA (1mg/ml) and luminol-hydrogen peroxide were prepared as described above. 10µl test compound of varying concentration (0.01-1mg/ml) was added to luminol-hydrogen peroxide (100µl, 1:10 dilution). 10µl HRP Type VIA or as a control 10µl Tris buffer was added. The light emission was measured on a Berthold Biolumat LB9500C.

The following test compounds were all screened in this manner. Chemical Abstracts Registry Numbers are in brackets:
- 1001: 4-(trimethylsilyl) benzeneboronic acid (17865-11-1)
- 1002: 1-naphthaleneboronic acid (31093-44-4)
- 1003: 5-bromo-2-methoxybenzeneboronic acid (84694-45-1)
- 1004: 2-biphenylboronic acid (4688-76-0)
- 1005: 2-hydroxy-5- [(3'-trifluoromethyl)-phenylazolbenzeneboronic acid
- 1006: 6-hydroxy-2- naphthaleneboronic acid
- 1007: 1-thianthreneboronic acid (108847-76-3)
- 1008: 4-dibenzofuranboronic acid (100124-06-9)
- 1009: 2-tolueneboronic acid (16419-60-6)
- 1010: 4-(phenoxy)benzeneboronic acid (109412-50-2)
- 1011: 4-biphenylboronic acid (5122-94-1)
- 1012: 2-phenyl-1,3,2-dioxaborinane (4406-77-3)
- 1013: bis(catechol)borate
- 1014: boraxarophenanthrene
- 1015: boroglycine
- 1016: tetraphenylboron sodium (143-66-8)
- 1017: pentaerythritol borate
- 1018: 4-(3'-borono-4'-hydroxyphenylazo)benzoic acid
- 1019: diphenylisobutoxyborane (23147-97-9)
- 1020: 2,4,6-trichlorophenylboronic acid (73852-18-3)
- 1021: 4-chloro-3-nitrophenylboronic acid
- 1022: 2,3-dichlorophenylboronic acid
- 1023: 2,5-dichlorophenylboronic acid (135145-90-3)
- 1024: 3,4-dichlorophenylboronic acid
- 1025: 3,5-dichlorophenylboronic acid (67492-50-6)
- 1026: 3-amino-2,4,6-trichlorophenylboronic acid
- 1027: 2-chlorophenylboronic acid
- 1028: 3-chlorophenylboronic acid (63503-60-6)
- 1029: 4-chlorophenylboronic acid (1679-18-1)
- 1030: 3-nitrophenylboronic acid (13331-27-6)
- 1031: 3-chloroacetylaminophenylboronic acid
- 1032: 3-(2'-methylbutylamino)phenylboronic acid
- 1033: 4-(4'-chloroanilino)phenylboronic acid
- 1034: 4-methylphenylboronic acid (5720-05-8)
- 1035: 1,4-phenyldiboronic acid
- 1036: dimethylphenylboronic acid (position of methyl groups not established)
- 1037: 4,4'-bis(phenylboronic acid) (4151-80-8)
- 1038: 4-(4'-bromophenyl)phenyl-di-n-butoxyborane
- 1039: di-(3',4',6'-trichlorophenoxy)-3,4,6-trichlorophenylborane
- 1040: di-(3',5'-dichlorophenoxy)-3,5-dichlorophenylborane
- 1041: 4-chlorophenyl-di-(4'-chlorophenoxy)borane
- 1042: 3-nitrophenylboronic acid, sodium salt
- 1043: 3-nitrophenylboronic acid, calcium salt
- 1044: 4-bromophenyl-di-n-butoxyborane
- 1045: 4-carboxy-3-nitrophenylboronic acid
- 1046: 2-benzimidazolylphenylboronic acid (58534-74-0)
- 1047: di-(1-naphthoxy)-l-naphthylborane
- 1048: diphenylboronic anhydride
- 1049: 2-boromethylphenyl-di-(2'-boromethylphenoxy)borane
- 1050: 2-(methylthiomethyl)phenylboronic acid
- 1051: methyl-(2-tolylboronic acid)sulfoxide

In Table 4, column (a) shows whether the test compound lowered the light output from the luminol-hydrogen peroxide solution (Y = yes, N = no). Column (b) shows whether the signal obtained after adding the HRP was greater in the presence of the test compound than in its absence, (Y = yes, N = no). A compound showing Y in column (b) is of preferred use in the present invention as it increases the light output of the reaction. Column (c) shows whether there was an increase in the signal:background ratio by over 25% of the control value (Y = yes, N = no), column (d) shows the actual calculated value of the signal:background ratio (value of column (b)/value of column (a)). Column (c) is a simplified representation of the results in column (d). Signal:background ratio is the ratio of light output in the presence and absence of HRP with the test compound being present.

All compounds increasing the signal:background ratio by over 25% of at least one concentration (Y in column (c)) are of use in the present invention. Those compounds that increase the light output (Y in column (b)) as well as increasing the signal:background ratio (Y in column (c)) are of especial use in the present invention. Thus, compounds 1001, 1002, 1003, 1005, 1006, 1009, 1010, 1011, 1012, 1013, 1015, 1017, 1018, 1019, 1021, 1022, 1024, 1026, 1028, 1029, 1030, 1033, 1034, 1036, 1037, 1038, 1040, 1041, 1044, 1045 and 1048 are of use in increasing the signal:background ratio of a chemiluminescent reaction. Of these, compounds 1001, 1005, 1011, 1015, 1021, 1029, 1033, 1037, 1038, 1041, 1044 and 1048 also increase the light output and are therefore of especial use in the present invention.

**TABLE 4**

| | | (a) | (b) | (c) | (d) |
|---|---|---|---|---|---|
| Compound | mg/ml | Decrease in background (without HRP with test cpd.) | Increase in signal (with HRP, with test cpd.) | Increase in signal: background ratio | Signal: Background: ratio (control = with no enhancer) |
| 1001 | 1 | Y | N | Y | 10.9 (2.1) |
| | 0.1 | Y | Y | Y | 6.7 |
| | 0.01 | Y | Y | Y | 3.2 |
| | | | | | |
| 1002 | 1 | Y | N | N | 0.7 (2.3) |
| | 0.1 | Y | N | Y | 3.4 |
| | 0.01 | Y | N | N | 2.5 |
| | | | | | |
| 1003 | 1 | Y | N | N | 2.6 (2.3) |
| | 0.1 | Y | N | Y | 12.1 |
| | 0.01 | Y | N | Y | 8.6 |
| | | | | | |
| 1004 | 1 | Y | N | N | 1.7 (2.3) |
| | 0.1 | Y | N | N | 2.2 |
| | 0.01 | Y | N | N | 1.9 |
| | | | | | |
| 1005 | 1 | Y | N | N | 2.4 (2.1) |
| | 0.1 | N | Y | Y | 2.9 |
| | 0.01 | N | Y | Y | 2.7 |
| | | | | | |
| 1006 | 1 | Y | N | N | 0.3 (1.8) |
| | 0.1 | Y | N | N | 1.5 |
| | 0.01 | Y | N | Y | 5.8 |
| | | | | | |
| 1007 | 1 | Y | N | N | 1.8 (2.1) |
| | 0.1 | Y | N | N | 2.5 |
| | 0.01 | Y | N | N | 2.1 |
| | | | | | |
| 1008 | 1 | Y | N | N | 1.3 (2.5) |
| | 0.1 | Y | N | N | 1.5 |
| | 0.01 | Y | N | N | 1.6 |
| | | | | | |
| 1009 | 1 | Y | N | N | 0.6 (1.8) |
| | 0.1 | Y | N | Y | 2.5 |
| | 0.01 | Y | N | N | 1.9 |
| | | | | | |
| 1010 | 1 | Y | N | Y | 5.0 (2.5) |
| | 0.1 | Y | N | Y | 4.6 |
| | 0.01 | Y | N | N | 3.0 |
| | | | | | |
| 1011 | 1 | Y | Y | Y | 48.7 (2.5) |
| | 0.1 | Y | Y | Y | >125 |
| | 0.01 | Y | Y | Y | >125 |
| | | | | | |
| 1012 | 1 | Y | N | Y | 17.1 (2.5) |
| | 0.1 | N | N | N | 2.2 |
| | 0.01 | N | Y | N | 2.2 |
| | | | | | |
| 1013 | 1 | Y | N | N | 1.2 (1.8) |
| | 0.1 | Y | N | Y | 3.1 |
| | 0.01 | Y | N | Y | 12.6 |
| | | | | | |
| 1014 | 1 | Y | N | N | 1.8 (2.5) |
| | 0.1 | Y | N | N | 1.6 |
| | 0.01 | Y | N | N | 2.3 |
| | | | | | |
| 1015 | 1 | Y | N | Y | 3.1 (2.1) |
| | 0.1 | Y | Y | Y | 2.9 |
| | 0.01 | Y | Y | N | 2.3 |
| | | | | | |
| 1016 | 1 | Y | N | N | 2.3 (1.9) |
| | 0.1 | Y | N | N | 2.0 |
| | 0.01 | Y | N | N | 2.0 |
| | | | | | |
| 1017 | 1 | Y | N | N | 1.8 (1.8) |
| | 0.1 | Y | Y | Y | 2.5 |
| | 0.01 | Y | N | N | 1.8 |
| | | | | | |
| 1018 | 1 | Y | N | Y | 4.2 (1.9) |
| | 0.1 | Y | N | Y | 24.5 |
| | 0.01 | Y | N | Y | 5.0 |
| 1019 | 1 | Y | N | N | |
| | 0.1 | Y | N | N | 3.0 (2.9) |
| | 0.01 | Y | N | Y | 3.2 (2.0) |
| | | | | | |
| 1020 | 1 | Y | N | N | |
| | 0.1 | Y | N | N | |
| | 0.01 | Y | N | N | 2.2 (2.0) |
| | | | | | |
| 1021 | 1 | Y | Y | Y | 247.9 (4.5) |
| | 0.1 | Y | Y | Y | 44.5 (2.8) |
| | 0.01 | Y | Y | Y | 2.3 (1.8) |
| | | | | | |
| 1022 | 1 | Y | N | N | |
| | 0.1 | Y | N | Y | 3.6 (2.7) |
| | 0.01 | Y | N | N | 2.2 (2.0) |
| | | | | | |
| 1023 | 1 | Y | N | N | |
| | 0.1 | Y | N | N | 3.1 (2.7) |
| | 0.01 | Y | N | N | |
| | | | | | |
| 1024 | 1 | Y | N | Y | 7.6 (4.5) |
| | 0.1 | Y | Y | Y | 5.4 (2.8) |
| | 0.01 | Y | N | N | |
| | | | | | |
| 1025 | 1 | Y | N | N | |
| | 0.1 | Y | N | N | |
| | 0.01 | Y | N | N | 2.1 (2.0) |
| | | | | | |
| 1026 | 1 | Y | N | Y | 40.5 (4.5) |
| | 0.1 | Y | Y | Y | 72.4 (2.8) |
| | 0.01 | Y | N | N | 2.1 (2.0) |
| | | | | | |
| 1027 | 1 | Y | N | N | |
| | 0.1 | Y | N | N | 3.2 (2.9) |
| | 0.01 | Y | N | N | 2.1 (2.0) |
| | | | | | |
| 1028 | 1 | Y | N | Y | 20.0 (4.5) |
| | 0.1 | Y | Y | Y | 4.2 (2.9) |
| | 0.01 | Y | N | N | |
| | | | | | |
| 1029 | 1 | Y | Y | Y | 1242.4(4.5) |
| | 0.1 | Y | Y | Y | 315.0 (2.8) |
| | 0.01 | Y | Y | Y | 6.0 (1.8) |
| | | | | | |
| 1030 | 1 | Y | N | Y | 50.0 (4.5) |
| | 0.1 | Y | Y | Y | 12.5 (2.8) |
| | 0.01 | Y | N | N | |
| | | | | | |
| 1031 | 1 | Y | N | N | |
| | 0.1 | Y | N | N | 3.3 (2.9) |
| | 0.01 | Y | N | N | 2.1 (2.0) |
| | | | | | |
| 1032 | 1 | Y | N | N | |
| | 0.1 | Y | N | N | 3.4 (2.9) |
| | 0.01 | Y | N | N | |
| | | | | | |
| 1033 | 1 | Y | Y | Y | 173.9 (4.5) |
| | 0.1 | Y | Y | Y | 67.7 (2.8) |
| | 0.01 | Y | Y | Y | 4.1 (1.8) |
| | | | | | |
| 1034 | 1 | Y | N | N | |
| | 0.1 | Y | Y | Y | 41.2 (2.9) |
| | 0.01 | Y | N | Y | 3.6 (1.9) |
| | | | | | |
| 1035 | 1 | Y | N | N | |
| | 0.1 | Y | N | N | |
| | 0.01 | Y | N | N | 2.1 (2.0) |
| | | | | | |
| 1036 | 1 | Y | N | N | |
| | 0.1 | Y | N | Y | 7.6 (2.9) |
| | 0.01 | Y | N | Y | 2.6 (2.0) |
| | | | | | |
| 1037 | 1 | Y | N | N | |
| | 0.1 | Y | Y | Y | 503.3 (2.9) |
| | 0.01 | Y | Y | Y | 35.5 (1.9) |
| | | | | | |
| 1038 | 1 | Y | Y | Y | 903.8 (4.5) |
| | 0.1 0.01 | Y Y | Y Y | Y Y | 2118.5(2.9) 442.2 (1.8) |
| | | | | | |
| 1039 | 1 | Y | N | N | |
| | 0.1 | Y | N | N | 3.4 (2.9) |
| | 0.01 | Y | N | N | |
| | | | | | |
| 1040 | 1 | Y | N | Y | 4.7 (4.5) |
| | 0.1 | Y | N | Y | 3.9 (2.9) |
| | 0.01 | Y | N | N | |
| | | | | | |
| 1041 | 1 | Y | Y | Y | 2606.2(4.5) |
| | 0.1 | Y | Y | Y | 360.2 (2.8) |
| | 0.01 | Y | Y | Y | 6.3 (1.9) |
| | | | | | |
| 1042 | 1 | Y | N | N | 5.2 (4.5) |
| | 0.1 | Y | N | N | 3.6 (2.9) |
| | 0.01 | Y | N | N | 2.3 (2.0) |
| | | | | | |
| 1043 | 1 | Y | N | N | |
| | 0.1 | Y | N | N | 3.4 (2.9) |
| | 0.01 | Y | N | N | |
| | | | | | |
| 1044 | 1 | Y | N | Y | 147.1 (4.5) |
| | 0.1 | Y | Y | Y | 623.1 (2.7) |
| | 0.01 | Y | Y | Y | 38.5 (1.9) |
| | | | | | |
| 1045 | 1 | Y | N | Y | 68.5 (4.5) |
| | 0.1 | Y | Y | Y | 18.1 (2.8) |
| | 0.01 | Y | N | N | 2.2 (2.0) |
| | | | | | |
| 1046 | 1 | Y | N | N | |
| | 0.1 | Y | N | N | |
| | 0.01 | Y | N | N | |
| | | | | | |
| 1047 | 1 | Y | N | N | |
| | 0.1 | Y | N | N | |
| | 0.01 | Y | N | N | |
| | | | | | |
| 1048 | 1 | Y | N | Y | 100.0 (4.5) |
| | 0.1 | Y | Y | Y | 139.0 (2.7) |
| | 0.01 | Y | Y | Y | 3.1 (1.8) |
| | | | | | |
| 1049 | 1 | Y | N | N | |
| | 0.1 | Y | N | N | 3.2 (2.9) |
| | 0.01 | Y | N | N | |
| | | | | | |
| 1050 | 1 | Y | N | N | |
| | 0.1 | Y | N | N | 3.3 (2.9) |
| | 0.01 | Y | N | N | |
| | | | | | |
| 1051 | 1 | Y | N | N | |
| | 0.1 | Y | N | N | |
| | 0.01 | Y | N | N | |
| | | | | | |

### EXAMPLE 4

### Effect of different peroxidase enzymes on the PBBA enhancement of a chemiluminescent reaction

Stock solutions (1mg/ml in 0.1mol/l Tris buffer, pH 8.6) of horseradish peroxidase Type VII, Type VIII and Arthromyces ramosus peroxidase (Sigma) were prepared. The luminol-hydrogen peroxide reagent was prepared as described previously. Luminol-hydrogen peroxide reagent (100µl, 1:10 dilution), either 40µl of PBBA (0.01mg/ml in 0.1mol/l Tris buffer, pH 8.6) or as a control, 40µl of Tris buffer (0.1mol/l, pH 8.6), and 10µl of HRP Type VII (1:50,000 dilution, 2ng) were added to a cuvette. The reagents were mixed and the light emission was recorded after 5 minutes using a Berthold Biolumat LB9500C.

The above experiment was repeated and the HRP Type VII was replaced by HRP Type VIII (2ng) or Arthromyces ramosus peroxidase (20pg).

The measured light output and signal:background ratios are shown in Table 5. PBBA did not increase the level of light output with any of the peroxidases tested, but the signal to background ratio was improved in all cases due to the background reduction caused by PBBA.

**TABLE 5**

| | Signal Reagent with PBBA | Signal: Background | Signal Reagent no PBBA | Signal: Background |
|---|---|---|---|---|
| Blank | 27,820 | | 58,759 | |
| | | | | |
| HRP Type VII | 49,703 | 1.8 | 77,694 | 1.3 |
| | | | | |
| HRP Type VIII | 156,204 | 5.6 | 280,472 | 4.8 |
| | | | | |
| Arthromyces rasmosus | 58,208 | 2.1 | 84,071 | 1.4 |

### EXAMPLE 5

### Assay of peroxidase-antibody conjugate utilizing PBBA

Anti-mouse IgG (whole molecule) peroxidase conjugate (Sigma Chemical Co.) was diluted (1:5000) in 0.1 mol/l Tris buffer (pH 8.6). The luminol-hydrogen peroxide was prepared as described previously. The following reagents were added to a cuvette: 100µl of luminol-hydrogen peroxide (1:10 dilution), either 40µl of PBBA (0.01 mg/ml in 0.1 mol/l Tris buffer) or as a control, 40µl of Tris buffer (0.1 mol/l, pH 8.6), and different amounts of anti-mouse IgG (5µl, 10µl, 20µl, 30µl, 40µl). The reagents were mixed and the light emission was measured using a Berthold Biolumat LB9500C.

The results are shown in Table 6. In the presence of PBBA the light emission was increased and the signal to background ratio for the assay of the HRP conjugate was significantly improved.

**TABLE 6**

| Anti-mouse IgG-HRP (ml) | Signal with PBBA | Signal: Background | Signal no PBBA | Signal : Background |
|---|---|---|---|---|
| 0 | 18,832 | | 23,112 | |
| 5 | 27,665 | 1.5 | 17,858 | 0.8 |
| 10 | 32,849 | 1.7 | 27,458 | 1.2 |
| 20 | 68,994 | 3.7 | 29,850 | 1.3 |
| 30 | 407,087 | 21.6 | 32,417 | 1.4 |

### EXAMPLE 6

### Enhanced chemiluminescent anti-oxidant assay using PBBA

The luminol-peroxide reagent (100µl), PBBA (40µl, 1:100 dilution of 1 mg/ml) and HRP (10µl, 1:50,000 dilution of 1mg/ml stock) were mixed together and the light emission measured for 25 minutes. A sample of human serum (2µl, 1:10 dilution in 0.1 mol/l Tris buffer, pH 8.6) was added and the light emission measured for a further 75 minutes.

Table 7 shows that addition of serum quenched the light emission and that after a 34 minute lag the light emission returns to its original level.

**TABLE 7**

| | Time (minutes) | Light units | Time (minutes) | Light units |
|---|---|---|---|---|
| | 0 | 5 | 50 | 5 |
| | 10 | 16 | 60 | 15 |
| | 20 | 68 | 70 | 49 |
| | | | | |
| (serum added) → | 25 | 88 | 80 | 60 |
| | 26 | 3 | 90 | 81 |
| | 30 | 3 | 100 | 90 |

### EXAMPLE 7

### Effect of para-iodophenylboronic acid (PIBA) (5006) as an enhancer of a chemiluminescent reaction

The experiment of Example 1 was repeated using PIBA in place of PBBA. Various dilutions of PIBA (Cookson Chemicals Ltd., Southampton, UK, 1mg/ml stock in DMSO) in Tris buffer were used. Table 8 shows the effect PIBA has on the signal:background ratio of a chemiluminescent reaction measured after 1 minute.

**TABLE 8**

| 4-Iodophenylboronic acid (PIBA) enhancement of the HRP-luminol-peroxide reaction | | | |
|---|---|---|---|
| PIBA | Light emission at 1 minute | | |
| µg | Background | Signal | Signal:Background Ratio |
| 0 | 15,016 | 91,665 | 6.1 |
| 0.01 | 12,862 | 78,419 | 6.1 |
| 0.1 | 13,221 | 74,487 | 7.1 |
| 1 | 10,282 | 324,312 | 31.5 |
| 10 | 5,517 | 540,760 | 98 |
| 20 | 3,837 | 135,065 | 35.2 |

### EXAMPLE 8

### Use of an organoboron enhancer in a chemiluminescent assay for a) peroxide and b) luminol

### a) Effect of 4-biphenvlboronic acid on a chemiluminescent assay for peroxide

The assay reagent consisted of the following reagents: 10µl 4-biphenylboronic acid (1:20 dilution of a 1mg/ml stock in DMSO), 10µl horseradish peroxidase (1:500,000 dilution of a 1mg/ml stock Type VIA in 0.1 mol/l Tris buffer pH 8.6) and 100µl luminol (0.025 g/l in 0.1 mol/l Tris buffer pH 8.6). These were mixed together and the light emission measured. A 10µl sample of a hydrogen peroxide solution (dilutions of a stock, 31µl 30% w/v hydrogen peroxide/ml in Tris buffer) was added, the contents of the assay tube mixed and the light emission recorded. The results are summarised in Table 9.

From Table 9 it can be seen that there was a dose-dependent increase in light output up to 2.4µmoles of peroxide.

**TABLE 9**

| Enhanced chemiluminescent assay for peroxide | |
|---|---|
| Hydrogen peroxide µmoles | Light emission at 5 minutes |
| 0 | 525 |
| 0.00024 | 659 |
| 0.0024 | 1,500 |
| 0.024 | 40,682 |
| 0.24 | 584,638 |
| 2.4 | 620,551 |
| 24 | 339,291 |

### b) Effect of 4-biphenylboronic acid on a chemiluminescent assay for luminol

The assay reagent consisted of the following reagents: 10µl 4-biphenylboronic acid (1:20 dilution of a 1mg/ml stock in DMSO), 10µl horseradish peroxidase (1:500,000 dilution of a lmg/ml stock Type VIA in 0.1 mol/l Tris buffer pH 8.6) and 100µl hydrogen peroxide solution (31µl 30% w/v hydrogen peroxide diluted in 100ml Tris buffer). These were mixed together and the light emission measured. A 10µl sample of a luminol solution (dilutions of a 0.25g/l in 0.1 mol/l Tris buffer pH 8.6 stock) was added, the contents of the assay tube mixed and the light emission recorded. The results are summarised in Table 10.

From Table 10 it can be seen that there was a dose-dependent increase in light output at all concentrations of luminol tested.

**TABLE 10**

| Enhanced chemiluminescent assay for luminol | |
|---|---|
| Luminol µmoles | Light emission at 10 minutes |
| 0 | 54 |
| 0.00125 | 145 |
| 0.0125 | 620 |
| 0.125 | 5,161 |
| 1.25 | 55,873 |
| 12.5 | 358,184 |
| 125 | 834,089 |

### EXAMPLE 9

### Effect of 4-(2-carboxyethenyl)phenylboronic acid (CPA) (5007) as an enhancer of a chemiluminescent reaction

The experiment of Example 1 was repeated using CPA in place of PBBA. Various dilutions of CPA (Cookson Chemicals Ltd., Southampton, UK, 0.01-20 pg in 1 mg/ml Tris buffer, pH 8.6) were used. Table 11 shows the effect CPA had on the light output (signal) and signal to background ratio of a chemiluminescent reaction measured using an Amerlite microplate reader (Kodak Clinical Diagnostics, Amersham, UK). "Amerlite" is a Registered Trade Mark.

**TABLE 11**

| CPA (µg) | Signal (with HRP) | Signal (no HRP) | Signal : Background ratio |
|---|---|---|---|
| 0 | 0.37 | 0.088 | 4.2 |
| 0.01 | 0.41 | 0.094 | 4.4 |
| 0.02 | 0.46 | 0.09 | 5.1 |
| 0.05 | 0.68 | 0.09 | 7.6 |
| 0.1 | 1.23 | 0.09 | 14.2 |
| 0.2 | 2.37 | 0.08 | 28.2 |
| 0.5 | 4.57 | 0.07 | 63.4 |
| 1.0 | 5.58 | 0.06 | 93.0 |
| 10 | 2.49 | 0.02 | 124.9 |
| 20 | 0.88 | 0.003 | 43.9 |

## Claims

1. A method of increasing the light output and/or signal: background ratio of light output from a chemiluminescent reaction of a dihydrophthalazinedione (DPD), a peroxidase enzyme catalyst and an oxidant, by carrying out this reaction in the presence of an enhancer, "signal" being in the presence of the peroxidase, "background" in its absence, characterised in that the enhancer comprises a compound of formula (I) 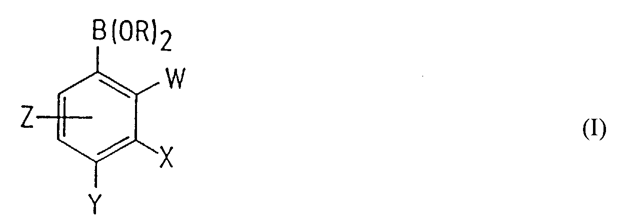 in which the R groups are the same and each is selected from hydrogen, n-butyl, 4'-chlorophenyl and 3',5'-dichlorophenyl; or the Rs together are O,O-propylene (thereby forming with the boron atom, a cyclic ether);
W is selected from hydrogen methyl, methoxy, hydroxy and chloro;
X is selected from hydrogen, chloro, amino and nitro;
Y is selected from hydrogen, methyl, carboxy, chloro, bromo, iodo, phenyl, phenoxy, 4'-chloroanilino, 4'-boronylphenyl, 4'-bromophenyl, 2'-carboxyethenyl and trimethylsilyl;
Z is selected from hydrogen, 5-chloro, 5-bromo, 5-(3'-trifluoromethyl)phenylazo and 6-chloro; or
W and X together may represent a fused benzene ring and X and Y together may represent a fused benzene ring substituted by hydroxy in the 6-position of the naphthalene ring numbering, provided that
(1) when each R is hydrogen:
(a) W, X, Y, Z are each hydrogen; or
(b) W, X and Z are each hydrogen and Y is selected from iodo, bromo, chloro, trimethylsilyl, phenoxy, phenyl, 4'-chloroanilino, methyl, 4'-boronylphenyl and 2'-carboxyethenyl; or
(c) W and Z are each hydrogen and:
(i) X and Y together represent a fused benzene ring substituted by hydroxy in the 6-position of the naphthalene ring numbering; or
(ii) X is either nitro or chloro and Y is chloro; or
(iii) X is nitro and Y is carboxy; or
(d) W, Y and Z are each hydrogen and X is amino, chloro or nitro; or
(e) W and X together represent a fused benzene ring and Y and Z are each hydrogen; or
(f) X and Y are each hydrogen and :
(i) W is methoxy and Z is 5-bromo; or
(ii) W is hydroxy and Z is 5-(3'-trifluoromethyl)phenylazo; or
(iii) W is methyl and Z is hydrogen; or
(g) W is chloro, X is chloro and Y and Z are each hydrogen; or
(h) W and Y are each chloro, X is amino and Z is 6-chloro;
(2) when each R is n-butyl, W, X and Z are each hydrogen and Y is bromo or 4'-bromophenyl;
(3) when each R is 4'-chlorophenyl, W, X and Z are each hydrogen and Y is chloro;
(4) when each R is 3',5'-dichlorophenyl, W and Y are each hydrogen, X is chloro and Z is 5-chloro; and
(5) when the Rs together represent O,O-propylene, X, Y and Z are each hydrogen;
or a compound selected from bis(catechol) borate, boroglycine, pentaerythritol borate, 4-(3'-borono-4'-hydroxyphenylazo) benzoic acid, diphenylisobutoxyborane, diphenylboronic anhydride and dimethylphenylboronic acid.

2. A method according to Claim 1, wherein enhancer is para-iodophenylboronic acid, para-bromophenylboronic acid, 4-biphenylboronic acid, 4-(trimethylsilyl)benzeneboronic acid, 2-hydroxy-5-[(3'-trifluoromethyl)phenylazo]benzeneboronic acid, boroglycine, 4-chloro-3-nitrophenylboronic acid, 4-chlorophenylboronic acid, 4-(2'-carboxyethenyl)phenylboronic acid, 4-(4'-bromophenyl)phenyl-di-n-butoxyborane, 4-chlorophenyl-di(4'-chlorophenoxy)borane, 4,4'-bis(phenylboronic acid), diphenylboronic anhydride, 4-(4'-chloroanilino)phenylboronic acid or 4-bromophenyl-di-n-butoxyborane.

3. A method according to claim 1 or 2, wherein the peroxidase enzyme is free or conjugated to a ligand and the presence or amount of the peroxidase is determined from the presence or amount of light output.

4. A method according to any preceding claim, wherein the peroxidase is horseradish peroxidase.

5. A method according to Claim 4, wherein the horseradish peroxidase is in the form of the basic isoenzyme.

6. A method according to any preceding claim, wherein the oxidant is hydrogen peroxide.

7. A method according to any preceding claim, wherein the DPD is luminol.

8. A method according to any preceding claim, wherein the chemiluminescent reaction is carried out at a pH of from 7.5 to 9.

9. A method according to any preceding claim for use in diagnostic assay for peroxidase.

10. A kit for use in diagnostic assay comprising in separate containers:
a chemiluminescent dihydrophthalazinedione (DPD);
a peroxidase enzyme catalyst; and
an enhancer which increases the signal : background ratio of light output, "signal" being in the presence of the peroxidase, "background" in its absence, wherein the enhancer is any one of the enhancers in Claim 1.

11. A kit according to Claim 10, wherein the enhancer is para-iodophenylboronic acid, para-bromophenylboronic acid, 4-biphenylboronic acid, 4-(trimethylsilyl)benzeneboronic acid, 2-hydroxy-5-[(3'-trifluoromethyl)phenylazo]benzeneboronic acid, boroglycine, 4-chloro-3-nitrophenylboronic acid, 4-chlorophenyl-boronic acid, 4-(2'-carboxyethenyl)phenylboronic acid, 4,4'-(bromophenyl)phenyl-di-n-butoxyborane, 4-chlorophenyl-di-(4'-chlorophenoxy)borane, 4,4'-bis(phenylboronic acid), diphenylboronic anhydride, 4-(4'-chloroanilino)phenylboronic acid and 4-bromophenyl-di-n-butoxyborane.

12. A kit according to Claim 10 or 11, wherein the peroxidase is conjugated to a ligand.

13. A kit according to Claim 10, 11 or 12, wherein the peroxidase is horseradish peroxidase.

14. A kit according to Claim 13, wherein the horseradish peroxidase is in the form of the basic isoenzyme.

15. A kit according to any one of Claims 10 to 14, which further comprises an oxidant.

16. A kit according to Claim 15, wherein the oxidant is hydrogen peroxide.

17. A kit according to any one of Claims 10 to 16, wherein the DPD is luminol.

## Patentansprüche

1. Verfahren zur Erhöhung der Lichtausbeute und/oder des Signal:Hintergrund-Verhältnisses der Lichtausbeute von einer chemolumineszierenden Reaktion eines Dihydrophthalazindions (DPD), eines Peroxidaseenzymkatalysators und eines Oxidationsmittels durch Durchführen dieser Reaktion in Gegenwart eines Verstärkers, wobei "Signal" in Gegenwart der Peroxidase und "Hintergrund" in deren Abwesenheit ist, dadurch gekennzeichnet, daß der Verstärker eine Verbindung der Formel (I) 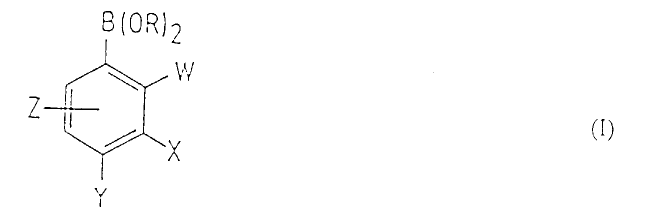 umfaßt, in der die Reste R gleich sind und jeweils ausgewählt sind aus Wasserstoff, n-Butyl, 4'-Chlorphenyl und 3',5'-Dichlorphenyl oder die Reste R zusammen 0,0-Propylen sind (wodurch zusammen mit dem Boratom ein cyclischer Ether gebildet wird),
W ausgewählt ist aus Wasserstoff, Methyl, Methoxy, Hydroxy und Chlor,
X ausgewählt ist aus Wasserstoff, Chlor, Amino und Nitro,
Y ausgewählt ist aus Wasserstoff, Methyl, Carboxy, Chlor, Brom, Iod, Phenyl, Phenoxy, 4'-Chloranilin, 4'-Boronylphenyl, 4'-Bromphenyl, 2'-Carboxyethenyl und Trimethylsilyl,
Z ausgewählt ist aus Wasserstoff, 5-Chlor, 5-Brom, 5-(3'-Trifluormethyl)phenylazo und 6-Chlor, oder
W und X zusammen für einen kondensierten Benzolring stehen können und X und Y zusammen für einen kondensierten Benzolring stehen können, der in 6-Stellung der Numerierung des Naphthalinringes durch Hydroxy substituiert ist, mit der Maßgabe, daß
(1) wenn jedes R Wasserstoff ist,
(a) W, X, Y, Z jeweils Wasserstoff sind, oder
(b) W, X und Z jeweils Wasserstoff sind und Y ausgewählt ist aus Iod, Brom, Chlor, Trimethylsilyl, Phenoxy, Phenyl, 4'-Chloranilin, Methyl, 4'-Boronylphenyl und 2'-Carboxyethenyl, oder
(c) W und Z jeweils Wasserstoff sind und
(i) X und Y zusammen für einen kondensierten Benzolring stehen, der in 6-Stellung der Numerierung des Naphthalinringes durch Hydroxy substituiert ist, oder
(ii) X entweder Nitro oder Chlor und Y Chlor ist oder
(iii) X Nitro und Y Carboxy ist, oder
(d) W, Y und Z jeweils Wasserstoff sind und X Amino, Chlor oder Nitro ist, oder
(e) W und X zusammen für einen kondensierten Benzolring stehen und Y und Z jeweils Wasserstoff sind, oder
(f) X und Y jeweils Wasserstoff sind, und
(i) W Methoxy und Z 5-Brom ist, oder
(ii) W Hydroxy und Z 5-(3'-Trifluormethyl)phenylazo ist, oder
(iii) W Methyl und Z Wasserstoff ist, oder
(g) W Chlor ist, X Chlor ist und Y und Z jeweils Wasserstoff sind, oder
(h) W und Y jeweils Chlor sind, X Amino ist und Z 6-Chlor ist,
(2) wenn jedes R n-Butyl ist, W, X und Z jeweils Wasserstoff sind und Y Brom oder 4'-Bromphenyl ist,
(3) wenn jedes R 4'-Chlorphenyl ist, W, X und Z jeweils Wasserstoff sind und Y Chlor ist,
(4) wenn jedes R 3',5'-Dichlorphenyl ist, W und Y jeweils Wasserstoff sind, X Chlor ist und Z 5-Chlor ist, und
(5) wenn die Reste R zusammen für O,O-Propylen stehen, X, Y und Z jeweils Wasserstoff sind,
oder eine Verbindung, ausgewählt aus Bis(catechin)borat, Borglycin, Pentaerythritborat, 4-(3'-Borono-4'-hydroxyphenylazo)-benzoesäure, Diphenylisobutoxyboran, Diphenylboronsäureanhydrid und Dimethylphenylboronsäure.

2. Verfahren nach Anspruch 1, wobei der Verstärker p-Iodphenylboronsäure, p-Bromphenylboronsäure, 4-Biphenylboronsäure, 4-(Trimethylsilyl)benzolboronsäure, 2-Hydroxy-5-[(3'-trifluormethyl)-phenylazo]benzolboronsäure, Borglycin, 4-Chlor-3-nitrophenylboronsäure, 4-Chlorphenylboronsäure, 4-(2'-Carboxyethenyl)-phenylboronsäure, 4-(4'-Bromphenyl)phenyl-di-n-butoxyboran, 4-Chlorphenyl-di(4'-chlorphenoxy)boran, 4,4'-Bis(phenylboronsäure), Diphenylboronsäureanhydrid, 4-(4'-Chloranilin)phenylboronsäure oder 4-Bromphenyl-di-n-butoxyboran ist.

3. Verfahren nach Anspruch 1 oder 2, wobei das Peroxidaseenzym frei oder mit einem Liganden konjugiert ist und das Vorhandensein oder die Menge der Peroxidase aus dem Vorhandensein oder der Menge der Lichtausbeute bestimmt wird.

4. Verfahren nach einem der vorangehenden Ansprüche, wobei die Peroxidase Meerrettichperoxidase ist.

5. Verfahren nach Anspruch 4, wobei die Meerrettichperoxidase in Form des basischen Isoenzyms vorliegt.

6. Verfahren nach einem der vorangehenden Ansprüche, wobei das Oxidationsmittel Wasserstoffperoxid ist.

7. Verfahren nach einem der vorangehenden Ansprüche, wobei das DPD Luminol ist.

8. Verfahren nach einem der vorangehenden Ansprüche, wobei die chemolumineszierende Reaktion bei einem pH-Wert von 7,5 bis 9 durchgeführt wird.

9. Verfahren nach einem der vorangehenden Ansprüche zur Anwendung in einem Diagnoseassay für Peroxidase.

10. Kit zur Verwendung in einem Diagnoseassay, umfassend in getrennten Behältern:
ein chemolumineszierendes Dihydrophthalazindion (DPD),
einen Peroxidaseenzymkatalysator und
einen Verstärker, der das Signal:Hintergrund-Verhältnis der Lichtausbeute erhöht, wobei "Signal" in Gegenwart der Peroxidase und "Hintergrund" in deren Abwesenheit ist, wobei der Verstärker einer der Verstärker nach Anspruch 1 ist.

11. Kit nach Anspruch 10, wobei der Verstärker p-Iodphenylboronsäure, p-Bromphenylboronsäure, 4-Biphenylboronsäure, 4-(Trimethylsilyl)benzolboronsäure, 2-Hydroxy-5-[(3'-trifluormethyl)-phenylazo]benzolboronsäure, Borglycin, 4-Chlor-3-nitrophenylboronsäure, 4-Chlorphenylboronsäure, 4-(2'-Carboxyethenyl)phenylboronsäure, 4,4'-(Bromphenyl)phenyl-di-n-butoxyboran, 4-Chlorphenyl-di-(4'-chlorphenoxy)boran, 4,4'-Bis(phenylboronsäure), Diphenylboronsäureanhydrid, 4-(4'-Chloranilin)phenylboronsäure und 4-Bromphenyl-di-n-butoxyboran ist.

12. Kit nach Anspruch 10 oder 11, wobei die Peroxidase mit einem Liganden konjugiert ist.

13. Kit nach Anspruch 10, 11 oder 12, wobei die Peroxidase Meerrettichperoxidase ist.

14. Kit nach Anspruch 13, wobei die Meerrettichperoxidase in Form des basischen Isoenzyms vorliegt.

15. Kit nach einem der Ansprüche 10 bis 14, umfassend ferner ein Oxidationsmittel.

16. Kit nach Anspruch 15, wobei das Oxidationsmittel Wasserstoffperoxid ist.

17. Kit nach einem der Ansprüche 10 bis 16, wobei das DPD Luminol ist.

## Revendications

1. Procédé pour augmenter l'émission lumineuse et/ou le rapport "signal" : "background" de l'émission lumineuse d'une réaction chimioluminescente entre une dihydrophtalazinedione (DPD), un catalyseur enzyme peroxydase et un oxydant, en effectuant cette réaction en présence d'un stimulateur, le "signal" étant observé en présence de la peroxydase, le "background" en son absence, caractérisé par le fait que le stimulateur comprend un composé de formule (I) 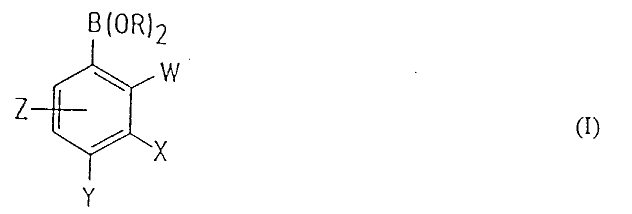 dans laquelle les groupes R sont identiques et sont chacun choisis parmi l'hydrogène, les groupes n-butyle, 4'-chlorophényle et 3',5'-dichlorophényle ; ou les R représentent ensemble l'O,O-propylène (formant ainsi un éther cyclique avec l'atome de bore) ;
W est choisi parmi l'hydrogène, les groupes méthyle, méthoxy, hydroxy et chloro ;
X est choisi parmi l'hydrogène, les groupes chloro, amino et nitro ;
Y est choisi parmi l'hydrogène, les groupes méthyle, carboxy, chloro, bromo, iodo, phényle, phénoxy, 4'-chloroanilino, 4'-boronylphényle, 4'-bromophényle, 2'-carboxyéthényle et triméthylsilyle ;
Z est choisi parmi l'hydrogène, les groupes 5-chloro, 5-bromo, 5-(3'-trifluorométhyl)phénylazo et 6-chloro ; ou
W et X peuvent représenter ensemble un cycle benzénique accolé et X et Y peuvent représenter ensemble un cycle benzénique accolé, substitué par un groupe hydroxy en position 6 du cycle naphtalénique, étant entendu que
(1) lorsque chaque R représente l'hydrogène :
(a) W, X, Y, Z sont chacun l'hydrogène ; ou
(b) W, X et Z sont chacun l'hydrogène et Y est choisi parmi les groupes iodo, bromo, chloro, triméthylsilyle, phénoxy, phényle, 4'-chloranilino, méthyle, 4'-boronylphényle et 2'-carboxyéthényle ; ou
(c) W et Z sont chacun l'hydrogène et :
(i) X et Y représentent ensemble un cycle benzénique accolé, substitué par un groupe hydroxy en position 6 du cycle naphtalénique ; ou
(ii) X est soit nitro, soit chloro et Y est chloro ; ou
(iii) X est nitro et Y est carboxy ; ou
(d) W, Y et Z sont chacun l'hydrogène et X est amino, chloro ou nitro ; ou
(e) W et X représentent ensemble un cycle benzénique accolé et Y et Z sont chacun l'hydrogène ; ou
(f) X et Y sont chacun l'hydrogène et :
(i) W est méthoxy et Z est 5-bromo ; ou
(ii) W est hydroxy et Z est 5-(3'-trifluorométhyl)phénylazo ; ou
(iii) W est méthyle et Z est l'hydrogène ; ou
(g) W est chloro, X est chloro et Y et Z sont chacun l'hydrogène ; ou
(h) W et Y sont chacun chloro, X est amino et Z est 6-chloro ;
(2) lorsque chaque R est n-butyle, W, X et Z sont chacun l'hydrogène et Y est bromo ou 4'-bromophényle ;
(3) lorsque chaque R est 4'-chlorophényle, W, X et Z sont chacun l'hydrogène et Y est chloro ;
(4) lorsque chaque R est 3',5'-dichlorophényle, W et Y sont chacun l'hydrogène, X est chloro et Z est 5-chloro ; et
(5) lorsque les R représentent ensemble l'O,O-propylène, X, Y et Z sont chacun l'hydrogène ;
ou un composé choisi parmi le borate de bis(catéchol), la boroglycine, le borate de pentaérythritol, l'acide 4-(3'-boro-4'-hydroxyphénylazo)benzoïque, le diphénylisobutoxyborane, l'anhydride diphénylborique et l'acide diméthylphénylborique.

2. Procédé selon la revendication 1, dans lequel le stimulateur est l'acide para-iodophénylborique, l'acide parabromophénylborique, l'acide 4-biphénylborique, l'acide 4-(triméthylsilyl)benzèneborique, l'acide 2-hydroxy-5-[(3'-trifluorométhyl)phénylazo]benzèneborique, la boroglycine, l'acide 4-chloro-3-nitrophénylborique, l'acide 4-chlorophénylborique, l'acide 4-(2'-carboxyéthényl)phénylborique, le 4-(4' -bromophényl)phényl-di-n-butoxyborane, le 4-chlorophényl-di-(4'-chlorophénoxy)borane, l'acide 4,4'-bis(phénylborique), l'anhydride diphénylborique, l'acide 4-(4'-chloranilino)phénylborique ou le 4-bromophényl-di-n-butoxyborane.

3. Procédé selon la revendication 1 ou 2, dans lequel l'enzyme peroxydase est libre ou conjuguée à un ligand et la présence ou la quantité de la peroxydase est déterminée à partir de la présence ou de la quantité d'une émission lumineuse.

4. Procédé selon l'une quelconque des revendications précédentes, dans lequel la peroxydase est la peroxydase de raifort.

5. Procédé selon la revendication 4, dans lequel la peroxydase de raifort est sous la forme de l'isoenzyme basique.

6. Procédé selon l'une quelconque des revendications précédentes, dans lequel l'oxydant est le peroxyde d'hydrogène.

7. Procédé selon l'une quelconque des revendications précédentes, dans lequel la DPD est le luminol.

8. Procédé selon l'une quelconque des revendications précédentes, dans lequel la réaction chimioluminescente est réalisée à un pH de 7,5 à 9.

9. Procédé selon l'une quelconque des revendications précédentes, destiné à être utilisé dans un test de diagnostic pour la peroxydase.

10. Trousse destinée à être utilisée dans un test de diagnostic, comprenant, dans des conteneurs séparés :
une dihydrophtalazinedione chimioluminescente (DPD) ;
un catalyseur enzyme peroxydase ; et
un stimulateur qui augmente le rapport "signal" : "background" de l'émission lumineuse, le "signal" étant en présence de la peroxydase, le "background" en son absence, le stimulateur étant l'un quelconque des stimulateurs de la revendication 1.

11. Trousse selon la revendication 10, dans laquelle le stimulateur est l'acide para-iodophénylborique, l'acide parabromophénylborique, l'acide 4-biphénylborique, l'acide 4-(triméthylsilyl)benzèneborique, l'acide 2-hydroxy-5-[(3'-trifluorométhyl)-phénylazo]benzèneborique, la boroglycine, l'acide 4-chloro-3-nitrophénylborique, l'acide 4-chlorophénylborique, l'acide 4-(2'-carboxyéthényl)phénylborique, le 4-(4'-bromophényl)phényl-di-n-butoxyborane, le 4-chlorophényl-di-(4'-chlorophénoxy)borane, l'acide 4,4'-bis(phénylborique), l'anhydride diphénylborique, l'acide 4-(4'-chloranilino)phénylborique et le 4-bromophényl-di-n-butoxyborane.

12. Trousse selon la revendication 10 ou 11, dans laquelle la peroxydase est conjuguée à un ligand.

13. Trousse selon la revendication 10, 11 ou 12, dans laquelle la peroxydase est la peroxydase de raifort.

14. Trousse selon la revendication 13, dans laquelle la peroxydase de raifort est sous la forme de l'isoenzyme basique.

15. Trousse selon l'une quelconque des revendications 10 à 14, qui comprend en outre un oxydant.

16. Trousse selon la revendication 15, dans laquelle l'oxydant est le peroxyde d'hydrogène.

17. Trousse selon l'une quelconque des revendications 10 à 16, dans laquelle la DPD est le luminol.
